# EUROPEAN PATENT SPECIFICATION

(11) **EP 4 066 735 B1**
(45) Date of publication and mention of the grant of the patent: **24.09.2025**
(21) Application number: 20901706.0
(22) Date of filing: 30.10.2020
(51) Int. Cl.: A61B 5/022, A61B 5/021, A61B 5/00

(54) **BLOOD PRESSURE MEASUREMENT APPARATUS**
BLUTDRUCKMESSGERÄT
APPAREIL DE MESURE DE TENSION ARTÉRIELLE

(30) Priority: 20.12.2019 CN 201911324782
(43) Date of publication of application: 05.10.2022
(73) Proprietor: Huawei Technologies Co., Ltd., Shenzhen, Guangdong 518129, (CN)
(72) Inventor: KUANG, Yunsheng, Shenzhen, Guangdong 518129 (CN)
(74) Representative: Gill Jennings & Every LLP
(86) International application number: PCT/CN2020/125381
(87) International publication number: WO 2021/120889

(56) References cited:
- WO-A1-2009/005933
- CN-A- 101 947 109
- CN-A- 109 199 352
- CN-U- 201 602 766
- CN-U- 201 822 850
- CN-U- 205 514 570
- JP-A- 2009 297 222
- JP-A- 2011 212 159
- JP-A- 2012 147 995
- US-A1- 2011 112 412
- US-A1- 2013 102 910

## Description

This application claims priority to Chinese Patent Application No. 2019113247823, filed with the China National Intellectual Property Administration on December 20, 2019 and entitled "AIRBAG, BLOOD PRESSURE MEASUREMENT APPARATUS, AND BLOOD PRESSURE MEASUREMENT METHOD.

### TECHNICAL FIELD

This application relates to the field of blood pressure measurement technologies, and in particular, to a blood pressure measurement apparatus.

### BACKGROUND

As living standard continuously improves, people pay more attention to their own health. Blood pressure, as a main physiological indicator of a human body, is also increasingly valued by people. Currently, product forms of sphygmomanometers on the market are mainly an upper-arm electronic sphygmomanometer and a wrist electronic sphygmomanometer. The two types of sphygmomanometers can perform single blood pressure measurement, but cannot perform dynamic blood pressure monitoring, and especially, blood pressure monitoring at night is difficult to perform. Therefore, miniaturization and wearableness of a sphygmomanometer become a trend, and a blood pressure watch emerges accordingly.

The blood pressure watch usually includes a watch core, a watch strap, an airbag, an air pump, and a barometric pressure sensor. Both the airbag and the watch strap may be sleeved on a wrist. Both the air pump and the barometric pressure sensor are disposed in the watch core. The air pump is configured to inflate the airbag. The barometric pressure sensor is configured to collect a pressure signal in the airbag to obtain a pulse wave signal, to implement blood pressure measurement.

However, a conventional blood pressure watch has different blood pressure measurement accuracy for different people. This is mainly reflected in different measurement accuracy for people with different wrist circumferences, and causes difficulty to cover a wider range of users.

Document US 2013/0102910 A1 discloses a cuff for a blood pressure meter, the cuff being adjustable in length.

### SUMMARY

An objective of embodiments of this application is to provide an airbag, a blood pressure measurement apparatus, and a blood pressure measurement method, to resolve a technical problem that a blood pressure watch has different blood pressure measurement accuracy for people with different wrist circumferences. The invention is defined by the independent claim. Advantageous examples are set out in the dependent claims.

To achieve the foregoing objective, the technical solutions used in embodiments of this application are as follows:

According to a first aspect, an embodiment of this application provides a blood pressure measurement apparatus comprising an airbag, a cavity, a pressing part, an air pump, and a barometric pressure sensor, wherein the airbag is long-strip-shaped, and wherein the airbag comprises: a plurality of grooves (101), the plurality of grooves (101) are distributed on a surface of the airbag along a length extension direction of the airbag, the grooves extend along a width direction of the airbag, each of the grooves is configured to divide the airbag into a pressurized area and a non-pressurized area along the length direction of the airbag when an external structure is pressed against the groove, the pressurized area and the non-pressurized area isolate air flow from each other; air holes distributed between every two adjacent grooves, wherein the air holes are used to connect the inside and the outside of the airbag; and blocking parts for blocking the air holes, wherein the airbag has a first surface and a second surface that are disposed back-to-back, the second surface is configured to be in contact with skin, each groove is disposed on the first surface, and each air hole is disposed on the first surface, wherein two air holes are distributed between every two adjacent grooves, one air hole is configured to be connected with an air pump, the other air hole is configured to be connected with a barometric pressure sensor, each groove is disposed on the first surface, each air hole is disposed on the first surface, and the two air holes between two adjacent grooves are disposed in parallel along the width direction of the airbag, wherein the airbag has a fixed end and a free end, the fixed end is fixed in the cavity, and the free end can be drawn out of the cavity; the pressing part is movably disposed in the cavity and can be pressed against the groove to divide the airbag into a pressurized area and a non-pressurized area, wherein the pressurized area is close to the free end; and the air pump is configured to be connected with the air hole to inflate and pressurize the pressurized area, and the barometric pressure sensor is configured to be connected with the air hole to collect a pressure signal in the pressurized area in real time, to obtain a pulse wave signal.

According to the airbag provided in this embodiment of this application, the plurality of grooves are distributed in the length direction of the airbag, and each groove 101 is configured to divide the airbag into the pressurized area and the non-pressurized area along the length direction of the groove 101 when the external structure is pressed against the groove. In this way, the external structure may be pressed against a groove at a different location, to adjust a length of the pressurized area, so as to adjust ratios of wrist circumferences of different people to the length of the pressurized area to proper ratios. This improves blood pressure signal collection stability and blood pressure measurement accuracy. In addition, there is no need to design airbags with different lengths for people with different wrist circumferences. This reduces costs of the airbag and a blood pressure measurement apparatus. After a wrist circumference of a user changes, there is no need to replace the airbag and the blood pressure measurement apparatus. This provides good experience and better blood pressure measurement accuracy for the user.

In a possible embodiment, the airbag may be made of an elastic material such as silica gel, polyvinyl chloride, or polyurethane elastomer rubber.

In a possible embodiment, the airbag has a first surface and a second surface that are disposed back-to-back, the second surface is disposed to be in contact with skin, each groove is recessed on the first surface and/or the second surface, and each air hole is disposed on the first surface and/or the second surface.

In a possible embodiment, two air holes are distributed between every two adjacent grooves, one air hole is configured to communicate with an air pump, and the other air hole is configured to communicate with a barometric pressure sensor. Each groove is recessed on the first surface, each air hole is disposed on the first surface, and the two air holes between two adjacent grooves are disposed in parallel along the width direction of the airbag.

In a possible embodiment, the airbag sequentially includes a base section, an adjustable section, and a connection section along the length direction of the airbag, the grooves are distributed on the adjustable section, a groove located at an edge of one side of the adjustable section is a boundary between the adjustable section and the base section, and a groove located at an edge of the other side of the adjustable section is a boundary between the adjustable section and the connection section.

In a possible embodiment, the grooves are distributed at equal intervals along a length direction of the adjustable section.

Alternately, the grooves are distributed along a length direction of the adjustable section in a manner in which the grooves are sparse at two ends and dense in the middle.

In a possible embodiment, a length of the base section is from 120 mm to 140 mm, a length of the adjustable section is from 90 mm to 180 mm, and 3 to 10 grooves are evenly distributed on the adjustable section.

In a possible embodiment, a sealed cavity is disposed at an inner side of the airbag facing the air hole, the blocking part is disposed in the sealed cavity, an outer peripheral wall of the blocking part fits an inner peripheral wall of the sealed cavity, and a spring is connected between the blocking part and the sealed cavity.

In a possible embodiment, an outer peripheral wall of the blocking part and an inner peripheral wall of the sealed cavity are both in a serrated shape and form a concave-convex fit with each other.

In a possible embodiment, the blocking parts are made of hard plastic or hard rubber.

According to the blood pressure measurement apparatus provided in this embodiment of this application, the pressing part, the air pump, and the barometric pressure sensor are disposed. In this way, the pressing part may be pressed against a groove at a different location on the airbag, to divide the airbag into the pressurized area and the non-pressurized area and adjust a length of the pressurized area, so as to adjust ratios of wrist circumferences of different people to the length of the pressurized area to proper ratios. This improves blood pressure signal collection stability and blood pressure measurement accuracy. In addition, the cavity is disposed, so that the airbag can be accommodated in the cavity when the airbag is not used, to prevent the airbag from being damaged. In addition, when the blood pressure measurement apparatus is a blood pressure watch, after the airbag is accommodated in the cavity, the blood pressure watch can be used and comfortably worn as an ordinary watch.

In a possible embodiment, the pressing part is plate-shaped, a first section of the groove is V-shaped, the first section is parallel to a length direction of the airbag, and is perpendicular to a width direction of the airbag, and the pressing part can be pressed against a bottom of the groove from top to bottom, so that the airbag is sealed by pressing at the groove to isolate air flow of the pressurized area from that of the non-pressurized area. The groove is disposed in a V shape, and the V shape has a tip. This ensures that the pressing part can be fully pressed against the bottom of the groove, to ensure sealing of the pressurized area. To be specific, it is ensured that at least in an atmospheric pressure range below 300 mm Hg, no airflow flows from the pressurized area to the non-pressurized area.

In a possible embodiment, a rotating shaft is disposed in the cavity, the fixed end is fixed on the rotating shaft, and the rotating shaft is rotatable to wind the airbag around the rotating shaft. The airbag is wound around the rotating shaft, to reduce space occupied by the airbag and the non-pressurized area.

In a possible embodiment, the cavity includes an accommodating part and an extension part connected to each other, an inner diameter of the accommodating part is greater than an inner diameter of the extension part, the rotating shaft is disposed in the accommodating part, the air pump and the barometric pressure sensor are disposed on an outer side of the extension part and penetrate the extension part, and the pressing part is disposed at a joint between the accommodating part and the extension part and functions as a gate of the accommodating part.

In a possible embodiment, in the length direction of the airbag, there is a first distance between a center of the pressing part and a center of a first air nozzle of the air pump and a second distance between a center of the groove and a center of an adjacent air hole, and the first distance is equal to the second distance.

In a possible embodiment, the blood pressure measurement apparatus further includes a fastener, the fastener is disposed on the extension part, the airbag penetrates the fastener, two positioning holes are disposed on a surface of the fastener, and the first air nozzle of the air pump and a second air nozzle of the barometric pressure sensor may respectively pass through the two positioning holes to be inserted into corresponding air holes of the airbag. The fastener is disposed to limit the airbag to some extent. This ensures that the air pump and the barometric pressure sensor are respectively aligned with the two air holes, and facilitates inflation, deflation, extraction, and pressure detection of the airbag. In addition, during movement of the airbag, the air holes and the positioning holes are stuck, to remind a user that the airbag is in a location for fixing.

In a possible embodiment, the blood pressure measurement apparatus further includes a lifting platform configured to lift or lower down the fastener and a corresponding part of the airbag. The fastener and the airbag are lowered down by using the lifting platform, so that the airbag is not limited by the first air nozzle and the second air nozzle when being drawn out. In addition, the fastener and the airbag are lifted by using the lifting platform, and the first air nozzle and the second air nozzle are respectively inserted into corresponding air holes in the lifting process.

In a possible embodiment, the blood pressure measurement apparatus further includes a control board, a first driving part, and a second driving part, the first driving part is configured to drive the rotating shaft to rotate, the second driving part is configured to drive the pressing part to move, and the control board is separately electrically connected to the first driving part, the second driving part, the air pump, the barometric pressure sensor, and the lifting platform.

In a possible embodiment, the blood pressure measurement apparatus further includes a watch core and a watch strap. The cavity, the air pump, the barometric pressure sensor, and the lifting platform are all disposed on the watch core, and the watch strap is disposed on two opposite sides of the watch core.

### BRIEF DESCRIPTION OF DRAWINGS

FIG. 1 is a schematic front view of a blood pressure measurement apparatus according to an embodiment of this application;
FIG. 2 is a schematic top view of a blood pressure measurement apparatus according to an embodiment of this application;
FIG. 3 is a schematic front view of an airbag according to an embodiment of this application;
FIG. 4 is a sectional view of a blocked air hole in FIG. 1;
FIG. 5 is a sectional view of an open air hole in FIG. 1;
FIG. 6 is a schematic top view of an air hole, a sealed cavity, and a blocking part in FIG. 4;
FIG. 7 is a schematic diagram of a circuit of a blood pressure measurement apparatus according to an embodiment of this application; and
FIG. 8 is a schematic of a structure of a blood pressure measurement apparatus when the blood pressure measurement apparatus is a blood pressure watch according to an embodiment of this application.

Reference numerals in the drawings:
10-Airbag; 20-Cavity; 30-Pressing part; 40-Air pump; 50-Barometric pressure sensor; 60-Rotating shaft; 70-Fastener; 80-Lifting platform; 90-Control board; 100-First driving part; 120-second driving part; 130-Watch face; 140-Watch core; 150-Watch strap; 101-Groove; 102-Air hole; 103-Blocking part; 104-Base section; 105-Adjustable section; 106-Connecting section; 107-First surface; 108-Second surface; 109-Sealed cavity; 110-Pressurized area; 111-Spring; 112-Connecting ring; 113-Fixed end; 114-Free end; 115-Non-pressurized area; 201-Accommodating part; 203-Extension part; 401-First air nozzle; 501-Second air nozzle.

### DESCRIPTION OF EMBODIMENTS

Embodiments of this application relate to an airbag, a blood pressure measurement apparatus, and a blood pressure measurement method (not claimed). The airbag can be sleeved on a wrist of a human, and can be inflated to press radial and ulnar arteries on the wrist of the human, so as to obtain a pulse wave signal. The blood pressure measurement apparatus may be worn on the wrist of the human, and can measure blood pressure of the human in real time, to implement dynamic blood pressure monitoring and blood pressure monitoring at night. The blood pressure measurement method is a method for measuring human blood pressure by using the foregoing blood pressure measurement apparatus.

The following briefly describes concepts in the embodiments above.

Blood pressure: Side pressure of blood on a blood vessel wall in a blood vessel. High or low blood pressure affects human health.

Radial artery: A lower part of the radial artery is covered only by the skin and fascia and is a site to palpate the pulse.

Styloid process of ulna: (styloid process of ulna), a human anatomy term, is a downward tapered prominence on a posteromedial side at the distal end of the ulna, and is palpable on the body surface.

Diastolic pressure: When the human heart relaxes, the arterial blood vessels elastically retract, a pressure generated is called diastolic pressure, also called lower pressure.

Systolic pressure: When the human heart contracts, the pressure in the arteries increases. In the middle of the contraction, the pressure in the arteries is the highest. In this case, the pressure of the blood on the inner wall of the blood vessel is called systolic pressure, also called high pressure.

According to experiments, a length of the airbag is a fixed value, and a coverage range of wrist circumferences of different people is between 130 mm and 220 mm. A span of the wrist circumference is relatively large, and the airbag of a single length cannot cover all people. Experiments show that a ratio of an airbag length to a wrist circumference directly affects stability of collecting a pulse wave signal feature and blood pressure measurement accuracy. If the ratio exceeds 1, that is, when the airbag length is greater than a wrist circumference of a measured person, the airbag inevitably overlaps on a wrist of the measured person. This seriously affects expansion of the airbag and obtaining of a pulse wave signal, and even an effective pulse wave signal cannot be obtained. If the ratio is small, that is, if the airbag length is insufficient to cover an effective part for obtaining the pulse wave signal, peak pressure of the obtained pulse wave signal shifts rightwards, and measured blood pressure is relatively high. In addition, a shift of peak pressure of the pulse wave signal causes instability of a pulse wave signal feature, and further reduces blood pressure measurement accuracy.

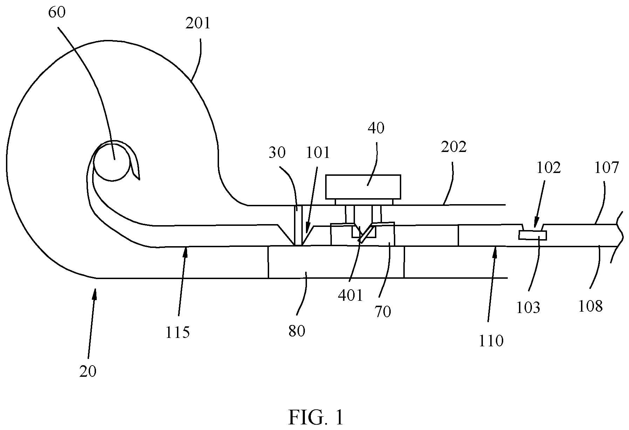

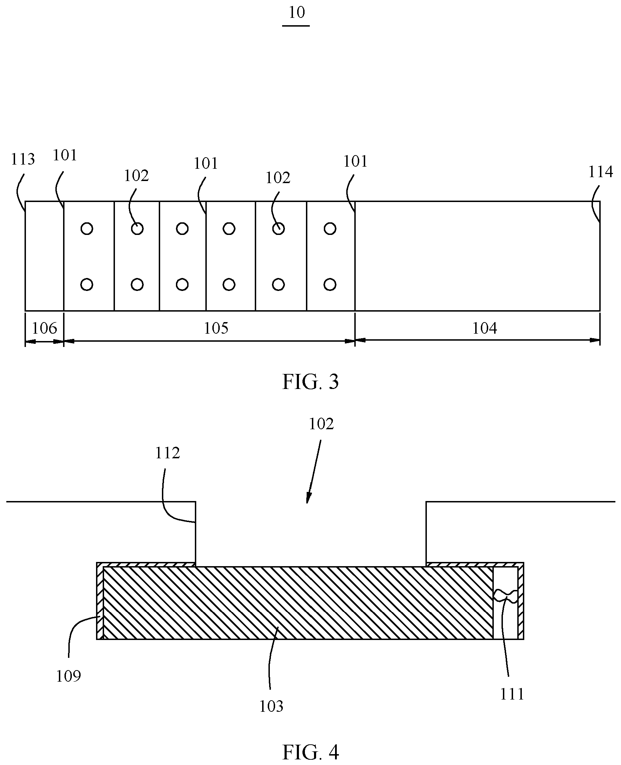

As shown in FIG. 1 to FIG. 3, an airbag 10 provided in embodiments of this application is described herein. The airbag 10 is used for blood pressure measurement. The airbag 10 is long-strip-shaped, a length extension direction of the airbag 10 is a left-right direction in FIG. 3, and a width direction of the airbag 10 is an up-down direction in FIG. 3. A plurality of grooves 101 are distributed on a surface of the airbag 10 along the length extension direction of the airbag 10, the grooves 101 extend along the width direction of the airbag 10, and each groove 101 is configured to divide the airbag 10 into a pressurized area 110 and a non-pressurized area 115 along the length direction of the airbag 10 when an external structure is pressed against the groove 101. The pressurized area 110 and the non-pressurized area 115 isolate air flow from each other, air holes 102 connecting the inside and the outside of the airbag 10 are distributed between every two adjacent grooves 101, and blocking parts 103 for blocking the air holes 102 are further disposed in the airbag 10.

Specifically, refer to FIG. 1. The airbag 10 is formed by sealing an upper surface layer and a lower surface layer. The upper and lower surface layers are stacked together when the airbag 10 is not inflated. When the airbag 10 is inflated, the airbag 10 begins to expand, and the two surface layers are separated from each other. In this case, the upper or lower surface layer abuts against radial and ulnar arteries. Air in the airbag 10 presses the radial and ulnar arteries, so that atmospheric pressure in the airbag 10 is synchronized with a pulse of the radial and ulnar arteries. When a barometric pressure sensor 50 collects a barometric pressure signal in the airbag 10, pulse wave signals of the radial and ulnar arteries can be obtained.

That each groove 101 is configured to divide the airbag 10 into a pressurized area 110 and a non-pressurized area 115 along the length direction of the airbag 10 when an external structure is pressed against the groove 101 means that each of the plurality of grooves 101 may be pressed by the external structure, but only one groove 101 is pressed at a time. When the external structure is pressed against the groove 101, the upper surface layer and the lower surface layer tightly abut against each other at a location of the groove 101. An area on one side of the groove 101 is set as the pressurized area 110, and an area on the other side of the groove 101 is set as the non-pressurized area 115. To be specific, the left side in FIG. 1 is the non-pressurized area 115, and the right side in FIG. 1 is the pressurized area 110. In other words, the grooves 101 function as scales of the airbag 10. Lengths of the pressurized area 110 and the non-pressurized area 115 change as the external structure is pressed against a groove 101 at a different location. Therefore, the length of the pressurized area 110 is adjustable. Pressurized areas 110 with different lengths may be adjusted based on wrist circumferences of different people, to ensure that a same airbag 10 can satisfy people with different wrist circumferences, and ratios of the length of the pressurized area 110 to wrist circumferences of different people can be adjusted to proper ratios. This improves blood pressure measurement accuracy.

The groove 101 penetrates the airbag 10 along the width direction of the airbag 10. When the external structure is fully pressed against the groove 101 along the width direction of the airbag 10, the pressurized area 110 and the non-pressurized area 115 can be fully isolated at least below an atmospheric pressure difference 300 mm Hg. To be specific, when the atmospheric pressure is less than 300 mm Hg, air in the pressurized area 110 cannot enter the non-pressurized area 115. This ensures sealing of the pressurized area 110, and improves blood pressure measurement accuracy.

Two air holes 102 are distributed between every two adjacent grooves 101. In this way, when any groove 101 is pressed, the pressurized area 110 has at least two air holes 102. The two air holes 102 are respectively configured to communicate with an air pump 40 and the barometric pressure sensor 50. The air pump 40 is configured to inflate the pressurized area 110, so that the pressurized area 110 can expand to press the radial and ulnar arteries. The barometric pressure sensor 50 is configured to connect to the pressurized area 110 to collect the barometric pressure signal in the pressurized area 110 in real time, to obtain a pulse wave signal and implement blood pressure measurement. Certainly, in another embodiment of this application, when the barometric pressure sensor 50 is integrated into the air pump 40, one air hole 102 may alternatively be distributed between every two adjacent grooves 101.

The blocking part 103 initially blocks the air hole 102, to seal the air hole 102 and prevent air leakage. When a first air nozzle 401 of the air pump 40 or a second air nozzle 501 of the barometric pressure sensor 50 presses against the blocking part 103, the blocking part 103 opens the air hole 102, the air pump 40 may inflate the pressurized area 110 through the air hole 102, and the barometric pressure sensor 50 may collect the barometric pressure signal in the pressurized area 110 through the air hole 102.

According to the airbag 10 provided in this embodiment of this application, the plurality of grooves 101 are distributed in the length direction of the airbag, and each groove 101 is configured to divide the airbag 10 into the pressurized area 110 and the non-pressurized area 115 along the length direction of the groove 101 when the external structure is pressed against the groove 101. In this way, the external structure may be pressed against a groove 101 at a different location, to adjust a length of the pressurized area 110, so as to adjust ratios of wrist circumferences of different people to the length of the pressurized area 110 to proper ratios. This improves blood pressure signal collection stability and blood pressure measurement accuracy. In addition, there is no need to design airbags 10 with different lengths for people with different wrist circumferences. This reduces costs of the airbag 10 and a blood pressure measurement apparatus. When a wrist circumference of a user changes, there is no need to replace the airbag 10 and the blood pressure measurement apparatus. This provides good experience and better blood pressure measurement accuracy for the user.

In a specific embodiment, the airbag 10 may be made of an elastic material such as silica gel, polyvinyl chloride, or polyurethane elastomer rubber.

In a specific embodiment, refer to FIG. 1 and FIG. 3. The airbag 10 has a first surface 107 and a second surface 108 that are disposed back-to-back. The second surface 108 is disposed to be in contact with skin, and each groove 101 is recessed on the first surface 107. In this way, when adjusting the length of the pressurized area 110, an external structure that is pressed against the groove 101 may be disposed on a same side of the airbag 10. Different grooves 101 are moved to the external structure for pressing against, to adjust the length of the pressurized area 110 without adjusting a location of the external structure. It can be understood that in another embodiment of this application, all the grooves 101 may be recessed on the second surface 108, or some of the grooves 101 are recessed on the first surface 107 and some of the grooves 101 are recessed on the second surface 108. This is not limited herein.

In a specific embodiment, refer to FIG. 1 and FIG. 3. Two air holes 102 are distributed between every two adjacent grooves 101. One air hole 102 is configured to communicate with the air pump 40, and the other air hole 102 is configured to communicate with the barometric pressure sensor 50. Each air hole 102 is disposed on the first surface 107, and the air holes 102 between every two adjacent grooves 101 are disposed in parallel along the width direction of the airbag 10. Distances between adjacent air holes 102 are the same along the length direction of the airbag 10. The air holes 102 are disposed on a same surface of the airbag 10. In this way, locations of the air pump 40 and the barometric pressure sensor 50 can be fixed. When the length of the pressurized area 110 is adjusted, the air holes 102 are moved to locations corresponding to the air pump 40 and the barometric pressure sensor 50 in sequence, and the air pump 40 and the barometric pressure sensor 50 do not need to be moved. In addition, two air holes 102 between two grooves 101 are disposed in parallel along the width direction. In this way, sizes occupied by the two air holes 102 in the length direction of the airbag 10 can be reduced as much as possible. Therefore, a distance between two adjacent grooves 101 is reduced as much as possible, that is, a distance between adjacent scales is reduced. This improves blood pressure measurement accuracy. It can be understood that in another embodiment of this application, all the air holes 102 may be disposed on the second surface 108, or some of the air holes 102 are disposed on the first surface 107 and some of the air holes 102 are disposed on the second surface 108. This is not limited herein.

In a specific embodiment, refer to FIG. 3. The airbag 10 sequentially includes a base section 104, an adjustable section 105, and a connection section 106 along the length direction of the airbag 10. Specifically, the base section 104, the adjustable section 105, and the connection section 106 are sequentially connected from right to left in FIG. 3. The grooves 101 are distributed on the adjustable section 105. A groove 101 located on an edge of one side of the adjustable section 105 is a boundary between the adjustable section 105 and the base section 104, and a groove 101 located on an edge of the other side of the adjustable section 105 is a boundary between the adjustable section 105 and the connection section 106. That is, the groove 101 located on the leftmost side and the groove 101 located on the rightmost side divide the entire airbag 10 into the base section 104, the adjustable section 105, and the connection section 106. One end of the airbag 10 may be fixed by disposing the connection section 106. In addition, because each person has a wrist circumference, even for a person with a thin wrist, the base section 104 may be of a value of a minimum wrist circumference obtained through data survey. No groove 101 is disposed in the base section 104 because the base section 104 is already of the minimum wrist circumference value, and the entire base section 104 needs to be used even if a small wrist circumference is measured. In other words, the base section 104 is disposed to reduce a distribution range of the grooves 101, and a manufacturing process and costs of the grooves 101. This can also reduce a length adjustment range for the user, and reduce adjustment difficulty for the user.

In a specific embodiment, refer to FIG. 3. The grooves 101 are distributed at equal intervals along a length direction of the adjustable section 105. That is, distances between every two adjacent grooves 101 are equal. In this way, each groove 101 functions as a scale on the adjustable section 105. The scales are even, so that users with different wrist circumferences can adjust the length of the pressurized area 110 to a most proper length, and a deviation is not large. This improves blood pressure signal collection stability and blood pressure measurement accuracy. It can be understood that, in another embodiment of this application, the grooves 101 may also be distributed along a length direction of the adjustable section 105 in a manner in which the grooves are sparse at two ends and dense in the middle. Because wrist circumferences of most people are concentrated, and there are fewer people with particularly large or particularly small wrist circumferences, the grooves 101 close to the middle may be distributed densely, and the grooves 101 close to the two ends may be distributed sparsely. In this way, adjustment precision of the adjustable section 105 of people with moderate wrist circumferences may be finer. This further improves blood pressure signal collection stability and blood pressure measurement accuracy.

According to statistics, wrist circumferences of different people range from 130 mm to 220 mm. That is, the minimum wrist circumference is 130 mm, and the maximum wrist circumference is 220 mm. Therefore, in this embodiment, a length of the base section 104 is set to 130 mm, a length of the adjustable section 105 is set to 90 mm, seven grooves 101 are evenly distributed on the adjustable section 105, and a distance between two adjacent grooves 101 is 15 mm. In this way, not only wrist circumference values within a range of 130 mm to 220 mm can be covered, but also a scale exists at every 15 mm between 130 mm and 220 mm. That is, a length adjustment deviation is about 7.5 mm. The length of the airbag 10 in this embodiment is set to be able to wrap around the wrist. To be specific, if there is a 7.5 mm deviation, it occurs on the back of the wrist, and does not affect measurement accuracy. This ensures measurement accuracy of different wrist circumferences. It can be understood that, in another embodiment of this application, lengths of the base section 104 and the adjustable section 105 may be properly adjusted based on an actual application requirement, and a distance between two adjacent grooves 101 may also be properly adjusted. For example, a length of the base section is any value from 120 mm to 140 mm; a length of the adjustable section 105 is any value from 90 mm to 180 mm; 3, 4, 5, 6, 8, 9, or 10 grooves 101 are evenly distributed on the adjustable section 105. This is not limited herein.

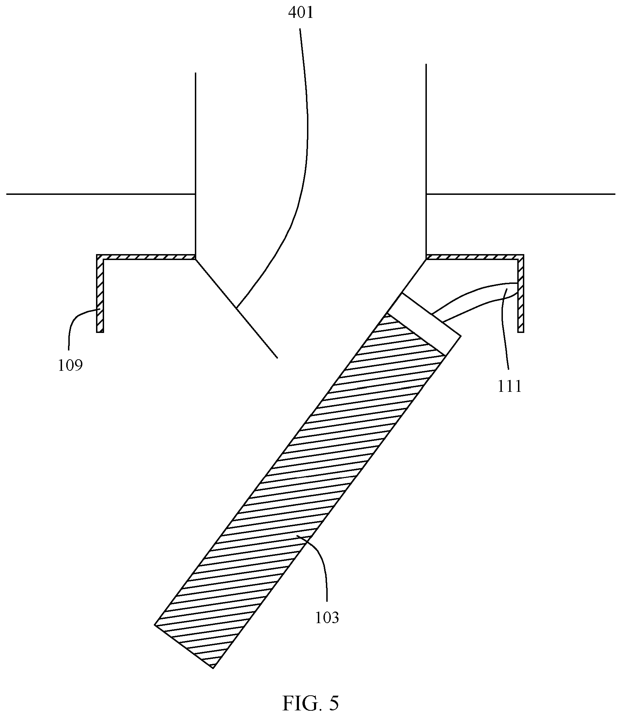

In a specific embodiment, refer to FIG. 4 and FIG. 5. A sealed cavity 109 is disposed at a location of an inner side of the airbag 10 facing the air hole 102, the sealed cavity 109 communicates with the air hole 102, the sealed cavity 109 is round, the sealed cavity 109 and the air hole 102 are disposed concentrically, and an inner diameter of the sealed cavity 109 is greater than an inner diameter of the air hole 102. The blocking part 103 is disposed in the sealed cavity 109. The blocking part 103 is cylindrical, and an outer peripheral wall of the blocking part 103 fits an inner peripheral wall of the sealed cavity 109, so that there is no gap between the blocking part 103 and the sealed cavity 109. To be specific, "the outer peripheral wall of the blocking part 103" herein is the outer peripheral wall of the blocking part 103 along a circumferential direction, and "the inner peripheral wall of the sealed cavity 109" is the inner peripheral wall of the sealed cavity 109 along the circumferential direction. A spring 111 is connected between the blocking part 103 and the sealed cavity 109, and one end of the blocking part 103 is connected to the inner peripheral wall of the sealed cavity 109 through the spring 111. When no force is applied to the spring 111, the blocking part 103 is in a closed state, and the blocking part 103 is accommodated in the sealed cavity 109 to block the air hole 102. When the first air nozzle 401 or the second air nozzle 501 is inserted into the corresponding air hole 102, the blocking part 103 in the air hole 102 is pushed away by the first air nozzle 401 or the second air nozzle 501, and a force is applied to the spring 111 connected to the blocking part 103. Because ends of the first air nozzle 401 and the second air nozzle 501 are funnel-shaped, after the first air nozzle 401 pushes away the blocking part 103, the first air nozzle 401 is connected to the airbag 10, and the air pump 40 may inflate and pressurize the airbag 10. When the first air nozzle 401 or the second air nozzle 501 is drawn out of the air hole 102, the blocking part 103 closes the air hole 102 under an effect of a restoring force of the spring 111.

To be specific, refer to FIG. 4. The sealed cavity 109 and the air hole 102 are disposed separately along an axial direction of the air hole 102, and the sealed cavity 109 and the air hole 102 are connected over a connection ring 112.

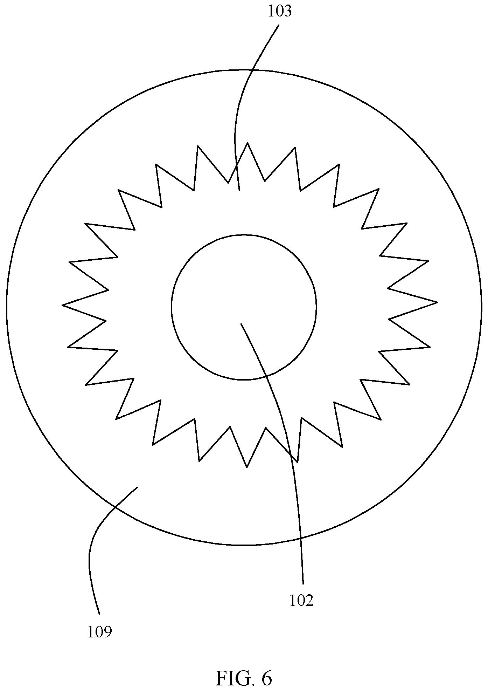

In a specific embodiment, refer to FIG. 6. Both the outer peripheral wall of the blocking part 103 and the inner peripheral wall of the sealed cavity 109 are in a serrated shape, and form a concave-convex fit with each other. This ensures connection tightness between the blocking part 103 and the sealed cavity 109, to improve air tightness of the airbag 10 and blood pressure measurement accuracy.

In a specific embodiment, the blocking part 103 is made of hard plastic or hard rubber. In this way, when the airbag 10 is inflated and expands under pressure, the blocking part 103 does not deform. This can ensure that air in the airbag 10 does not leak out from the corresponding air hole 102, to ensure air tightness of the airbag 10.

Based on a same application concept, an embodiment of this application further provides a blood pressure measurement apparatus. As shown in FIG. 1 to FIG. 3, the blood pressure measurement apparatus includes a cavity 20, a pressing part 30, the air pump 40, the barometric pressure sensor 50, and the airbag 10. The airbag 10 has a fixed end 113 and a free end 114. The fixed end 113 is fixed in the cavity 20, and the free end 114 extends from the cavity 20 and may be fixed on the back of the wrist after wrapping around the wrist. The pressing part 30 is movably disposed in the cavity 20 and can be pressed against the groove 101 to divide the airbag 10 into the pressurized area 110 and the non-pressurized area 115. The pressurized area 110 is close to the free end 114, the non-pressurized area 115 is close to the fixed end 113, and the non-pressurized area 115 is accommodated in the cavity 20. The pressurized area 110 may extend from the cavity 20 and may be wound around the wrist to press human radial and ulnar arteries. The air pump 40 is disposed outside the cavity 20 and is configured to communicate with an air hole 102 to inflate the pressurized area 110. The barometric pressure sensor 50 is disposed outside the cavity 20 and is configured to communicate with an air hole 102 to collect a pressure signal in the pressurized area 110 in real time to obtain a pulse wave signal. To be specific, two air holes 102 are disposed between two adjacent grooves 101, and the two air holes 102 are configured to respectively communicate with the air pump 40 and the barometric pressure sensor 50. The cavity 20 is configured to accommodate the non-pressurized area 115 of the airbag 10, and when the airbag 10 does not work, accommodate the entire airbag 10.

In specific use, the cavity 20 is first fixed on the back of the wrist, then the free end 114 of the airbag 10 is drawn out of the cavity 20, and the free end 114 is pulled from the back of the wrist to pass one side, the front, and the other side of the wrist in sequence, so that the airbag 10 wraps around the wrist, and the free end 114 is fixed on the back of the wrist from the other side. Then, the pressing part 30 is driven to press against a corresponding groove 101 on the back of the wrist, to divide the airbag 10 into the pressurized area 110 and the non-pressurized area 115. The pressing part 30 is set at a location, so that the pressurized area 110 can completely cover the front and the two sides of the wrist. Finally, the air pump 40 inflates the pressurized area 110, and the barometric pressure sensor 50 collects a pressure signal in the pressurized area 110 in real time to obtain a pulse wave signal. To be specific, when the blood pressure measurement apparatus is a blood pressure watch, because the cavity 20 is disposed on a watch core, when the blood pressure watch is worn on the wrist, the cavity 20 is fixed on the back of the wrist, and the free end 114 may also be fixed by pressing by a watch strap; or a fastener such as a hook may be disposed on the free end 114 to fix the free end on the watch core.

According to the blood pressure measurement apparatus provided in this embodiment of this application, the pressing part 30, the air pump 40, and the barometric pressure sensor 50 are disposed. In this way, the pressing part 30 may be pressed against a groove 101 at a different location on the airbag 10, to divide the airbag 10 into the pressurized area 110 and the non-pressurized area 115 and adjust a length of the pressurized area 110, so as to adjust ratios of wrist circumferences of different people to the length of the pressurized area 110 to proper ratios for measurement. This improves blood pressure signal collection stability and blood pressure measurement accuracy. In addition, the cavity 20 is disposed, so that the airbag 10 can be accommodated in the cavity 20 when the airbag 10 is not used, to prevent the airbag 10 from being damaged. In addition, when the blood pressure measurement apparatus is a blood pressure watch, after the airbag 10 is accommodated in the cavity 20, the blood pressure watch can be used and comfortably worn as an ordinary watch.

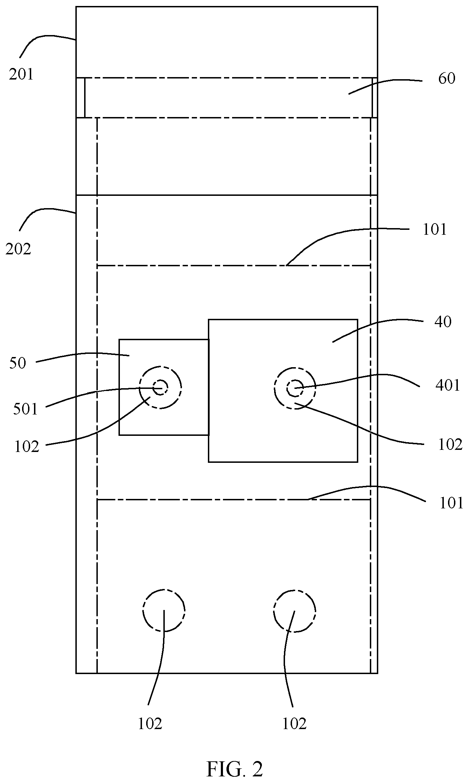

In a specific embodiment, refer to FIG. 1 and FIG. 2. A rotating shaft 60 is disposed in the cavity 20, the fixed end 113 is fixed on the rotating shaft 60, and the rotating shaft 60 may rotate to wind the airbag 10 around the rotating shaft 60. The rotating shaft 60 is connected to a first driving part 100. The first driving part 100 is disposed in the cavity 20 or disposed outside the cavity 20. The first driving part 100 can drive the rotating shaft 60 to rotate, and the first driving part 100 is electrically connected to a control board 90 in the blood pressure measurement apparatus. To be specific, the first driving part 100 is a stepper motor. In actual application, when the measurement ends and the airbag 10 is not needed, the control board 90 sends a control command to the first driving part 100, and the first driving part 100 drives the rotating shaft 60 to rotate in steps, to wind the airbag 10 around the rotating shaft 60 and accommodate the airbag 10 in the cavity 20. When the airbag 10 is needed, the fixed end 113 of the airbag 10 is manually pulled, and a part of the airbag 10 is unwound from the rotating shaft 60.

In a specific embodiment, refer to FIG. 1 and FIG. 2. The cavity 20 includes an accommodating part 201 and an extension part 202, and the accommodating part 201 and the extension part 202 are connected to each other along the length direction of the airbag 10. The accommodating part 201 is approximately cylindrical, an inner diameter of the accommodating part 201 is greater than an inner diameter of the extension part 202, and the rotating shaft 60 is disposed in the accommodating part 201. That is, the accommodating part 201 is configured to accommodate the airbag 10, and the inner diameter of the accommodating part 201 is set to be larger to accommodate the airbag 10. The air pump 40 and the barometric pressure sensor 50 are disposed on an outer side of the extension part 202 and penetrate the extension part 202, and the first air nozzle 401 and the second air nozzle 501 extend into the extension part 202. In this way, the air pump 40 and the barometric pressure sensor 50 may be connected to the airbag 10 through the air holes 102 located in the extension part 202. The pressing part 30 is disposed at a joint between the accommodating part 201 and the extension part 202 and functions as a gate of the accommodating part 201. That is, the pressing part 30 can be pressed against only a groove 101 at the joint between the accommodating part 201 and the extension part 202. In other words, the non-pressurized area 115 is located in the accommodating part 201, and the pressurized area 110 extends from the extension part 202.

Refer to FIG. 1. There is a first distance between a center of the pressing part 30 and a center of the first air nozzle 401 of the air pump 40 and a second distance between a center of the groove 101 and a center of an adjacent air hole 102, and the first distance is equal to the second distance. To be specific, after locations of the pressing part 30, the air pump 40, and the barometric pressure sensor 50 are fixed, as long as the pressing part 30 is pressed against one of the grooves 101, there are two air holes 102 facing the air pump 40 and the sensor, and the air nozzles only need to be inserted into the air holes 102.

In a specific embodiment, refer to FIG. 1 and FIG. 2. The pressing part 30 is plate-shaped, and the pressing part 30 is vertically disposed. The pressing part 30 extends along the width direction of the airbag 10, and a width of the pressing part 30 in the width direction of the airbag 10 is greater than or equal to the width of the airbag 10. In this way, the pressing part 30 can be fully pressed against the entire groove 101, to ensure sealing of the pressurized area 110. A first section of the groove 101 is V-shaped, the first section is parallel to the length direction of the airbag 10, and is perpendicular to the width direction of the airbag 10, and the pressing part 30 may be pressed against a bottom of the groove 101 from top to bottom, so that the airbag 10 is sealed by pressing at the groove 101, to isolate the air flow of the pressurized area 110 from that of the non-pressurized area 115. To be specific, when the pressing part 30 is pressed against the bottom of the groove 101 from top to bottom, the upper and lower surface layers of the airbag 10 abut against each other at the groove 101, so that the entire airbag 10 is divided into the pressurized area 110 and the non-pressurized area 115. In this embodiment, the groove 101 is disposed in a V shape, and the V shape has a tip. This ensures that the pressing part 30 can be fully pressed against the bottom of the groove 101, to ensure sealing of the pressurized area 110.

To be specific, the pressing part 30 is connected to a second driving part 120, and the second driving part 120 is configured to drive the pressing part 30 to move from top to bottom, so that the pressing part 30 is pressed against the groove 101. In addition, after the airbag 10 is accommodated in the accommodating part 201, the accommodating part 201 may be further isolated from the outside, to prevent the airbag 10 from getting out of the accommodating part 201.

In a specific embodiment, refer to FIG. 1. The blood pressure measurement apparatus further includes a fastener 70. The fastener 70 is disposed in the extension part 202, the airbag 10 penetrates the fastener 70, and the fastener 70 has a circumferential limiting function on the airbag 10. In other words, when the airbag 10 penetrates the fastener 70, the airbag 10 can move only along the length direction of the airbag 10. Two positioning holes (not shown in the figure) are disposed on a surface of the fastener 70. The two positioning holes are disposed in parallel along the width direction of the airbag 10, and a distance between centers of the two positioning holes is equal to a distance between centers of the two air holes 102. That is, when the airbag 10 moves, two air holes 102 are disposed facing the two positioning holes each time. Because the positioning holes and the air holes 102 are disposed, and a protrusion is disposed on an inner side of the fastener 70, when the air holes 102 pass the positioning holes, the air holes 102 and the positioning holes are stuck when the air holes 102 are in contact with the positioning holes because the protrusion blocks the airbag 10, to remind that the air holes 102 are aligned with the positioning holes. In this case, the pressing part 30 may be driven downward to press against the groove 101, and the first air nozzle 401 of the air pump 40 and the second air nozzle 501 of the barometric pressure sensor 50 may be driven to respectively pass through the two positioning holes to respectively insert into the two corresponding air holes 102 of the airbag 10. In this embodiment, the fastener 70 is disposed, so that the airbag 10 has a limiting and positioning function. This ensures that the air pump 40 and the barometric pressure sensor 50 are respectively aligned with the two air holes 102, and facilitates inflation, deflation, extraction, and pressure detection of the airbag 10.

In a specific embodiment, refer to FIG. 1. The blood pressure measurement apparatus further includes a lifting platform 80, and the lifting platform 80 is configured to lift or lower down the fastener 70 and a corresponding part of the airbag 10. To be specific, when not working, the lifting platform 80 is disposed outside a bottom of the extension part 202, and the fastener 70 is disposed on the lifting platform 80 and is located in the extension part 202. In this case, both the fastener 70 and the corresponding part of the airbag 10 are located at the bottom of the extension part 202. This facilitates drawing and winding of the airbag 10, and prevents the airbag 10 from interfering with the first air nozzle 401 and the second air nozzle 501. When the airbag 10 has been drawn out for a required length, the fastener 70 and the corresponding part of the airbag 10 may be lifted to an approximate middle height of the extension part 202 by using the lifting platform 80. In a process of lifting the airbag 10, the first air nozzle 401 and the second air nozzle 501 are respectively inserted into the corresponding air holes 102.

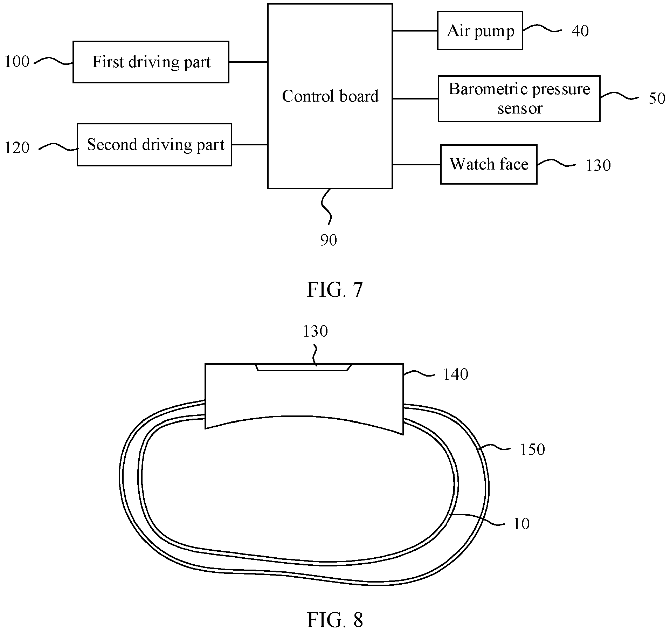

In a specific embodiment, refer to FIG. 7. The blood pressure measurement apparatus further includes the control board 90. The control board 90 is electrically connected to the first driving part 100, the second driving part 120, the air pump 40, the barometric pressure sensor 50, and the lifting platform 80. In this way, working sequences of the first driving part 100, the second driving part 120, the air pump 40, the barometric pressure sensor 50, and the lifting platform 80 can be separately controlled by the control board 90.

In a specific embodiment, refer to FIG. 8. The blood pressure measurement apparatus is a blood pressure watch, and further includes a watch core 140 and a watch strap 150. The cavity 20, the air pump 40, the barometric pressure sensor 50, and the lifting platform 80 are all disposed on the watch core 140. A watch face 130 is disposed on the watch core 140, and the watch face 130 is electrically connected to the control board 90. A blood pressure value measured by the barometric pressure sensor 50 may be displayed on the watch face 130, and the watch strap 150 is disposed on two opposite sides of the watch core 140.

Based on a same application concept, an embodiment of this application further provides a blood pressure measurement method. Blood pressure measurement is performed by using the foregoing blood pressure measurement apparatus, and the method includes the following steps.

The gate of the accommodating part 201 is opened.

When the airbag 10 is not used, the entire airbag 10 is wound around the fastener 70, the free end 114 of the airbag 10 stays on the fastener 70, the gate in the accommodating part 201 is closed by using the pressing part 30, and the pressing part 30 presses the free end 114 of the airbag 10, so that the free end 114 of the airbag 10 is fixed on the fastener 70. When the user wants to measure blood pressure, the pressing part 30 needs to be lifted, so that the user draws the airbag 10 out of the accommodating part 201 by hand.

The free end 114 of the airbag 10 is drawn out, and the free end 114 is pulled based on the wrist circumference until the airbag covers the styloid process of ulna on the outer side of the wrist. To be specific, a hook may be disposed at the free end 114 to fix the free end 114 on the watch core 140, or the free end 114 may be fixed by pressing the free end 114 by the watch strap 150.

Based on an experimental conclusion of a ratio of the length of the airbag 10 to the user's measured wrist circumference, an optimal ratio is between 0.8 and 0.85. That is, the airbag 10 needs to cover the front and the two sides of the wrist. In addition, a start location of the pressurized area 110 is located on the back of the wrist (the gate of the cavity 20 is a boundary between the pressurized area 110 and the non-pressurized area 115, and the gate is located in the watch core on the back of the wrist). Therefore, the tail of the pressurized area 110 of the airbag 10 should reach the styloid process of ulna of the wrist of the user. In this way, the pressurized area 110 of the airbag 10 can cover the front and the two sides of the wrist, so that a ratio of the length the pressurized area 110 to a wrist circumference reaches a proper ratio range. This ensures accuracy and stability of a pulse wave signal obtained by the barometric pressure sensor 50 during blood pressure measurement, to ensure blood pressure measurement accuracy.

The lifting platform 80 is driven to lift, so that the first air nozzle 401 of the air pump 40 and the second air nozzle 501 of the barometric pressure sensor 50 are inserted into corresponding air holes 102.

In this embodiment, the first surface 107 (a front surface) of the airbag 10 has parallel air holes 102 with six gears. When the user draws the airbag 10 out of the accommodating part 201 by hand and pull the free end to the styloid process of ulna on the outer side of the wrist, the airbag 10 passes through the fastener 70, and the air holes 102 on the upper surface of the airbag 10 are stuck, to remind the user that the airbag 10 is pulled to a location for fixing. In this case, the airbag 10 has been pulled out and fixed, and in a process in which the lifting platform 80 lifts, the first air nozzle 401 and the second air nozzle 501 are respectively inserted into the corresponding air holes 102.

The pressurized area 110 is inflated by using the air pump 40, and blood pressure is measured by using the barometric pressure sensor 50.

According to the foregoing steps, the airbag 10 is pulled to an optimal length, the air nozzles of the air pump 40 and the barometric pressure sensor 50 are inserted into the corresponding air holes 102 of the airbag 10, and preparation works before blood pressure measurement are completed. In this case, the air pump 40 can inflate and pressurize the airbag 10, to start a blood pressure measurement process. The air pump 40 inflates the airbag 10, to increase pressure inside the airbag 10, and the airbag 10 expands, and presses the radial and ulnar arteries at the wrist. The barometric pressure sensor 50 collects a pressure signal in the airbag 10 in real time. Because the radial and ulnar arteries are pressed, a pulse wave signal is superimposed on the signal collected by the barometric pressure sensor 50. The pulse wave signal is obtained from the original signal, features (including a peak pressure, a maximum slope point, and the like) of the pulse wave signal are calculated, and final diastolic pressure and systolic pressure values are calculated by using a blood pressure model.

The measurement ends, the air pump 40 is powered off and connected to the atmosphere, and the airbag 10 is deflated by using the air pump 40.

The air pump 40 not only has a function of inflating the airbag 10, but also has a function of extracting air from the airbag 10. When the blood pressure measurement ends, the air pump 40 deflates the pressurized area 110 of the airbag 10. However, after the deflation, some air remains in the pressurized area 110 of the airbag 10. If this air is not extracted, in a process of winding the airbag 10 into the accommodating part 201, because the air nozzles of the air pump 40 and the barometric pressure sensor 50 are drawn out of the air holes 102, and the airbag 10 is sealed, the airbag 10 cannot be wound into the accommodating part 201. Therefore, before winding, the air pump 40 needs to extract the air remaining in the pressurized area 110 of the airbag 10. After the air is extracted, the air nozzles of the air pump 40 and the barometric pressure sensor 50 are drawn out of the air holes 102.

The pressing part 30 is lifted, the lifting platform 80 is driven to lower down, and the rotating shaft 60 is started to rotate to wind the airbag 10 into the cavity 20.

It can be understood that driving action instructions in the foregoing steps are all sent by the control board 90 and completed by the foregoing corresponding parts.

## Claims

1. A blood pressure measurement apparatus comprising an airbag (10), a cavity (20), a pressing part (30), an air pump (40), and a barometric pressure sensor (50), wherein the airbag (10) is long-strip-shaped, and wherein the airbag (10) comprises:
a plurality of grooves (101), the plurality of grooves (101) are distributed on a surface of the airbag (10) along a length extension direction of the airbag (10), the grooves extend along a width direction of the airbag (10), each of the grooves is configured to divide the airbag (10) into a pressurized area (110) and a non-pressurized area (115) along the length direction of the airbag (10) when an external structure is pressed against a groove, the pressurized area and the non-pressurized area (115) isolate air flow from each other;
air holes (102) distributed between every two adjacent grooves, wherein the air holes are used to connect the inside and the outside of the airbag (10); and
blocking parts (103) for blocking the air holes,
wherein the airbag (10) has a first surface (107) and a second surface (108) that are disposed back-to-back, the second surface (108) is configured to be in contact with skin, each groove is disposed on the first surface (107), and each air hole is disposed on the first surface (107),
**characterized in that**
two air holes are distributed between every two adjacent grooves, one air hole is configured to be connected with the air pump (40), the other air hole is configured to be connected with the barometric pressure sensor (50), each groove is disposed on the first surface (107), each air hole is disposed on the first surface (107), and the two air holes between two adjacent grooves are disposed in parallel along the width direction of the airbag (10), wherein
the airbag (10) has a fixed end and a free end, the fixed end is fixed in the cavity (20), and the free end can be drawn out of the cavity (20);
the pressing part (30) is movably disposed in the cavity (20) and can be pressed against the groove to divide the airbag (10) into a pressurized area and a non-pressurized area (115), wherein the pressurized area is close to the free end; and
the air pump (40) is configured to be connected with the one air hole to inflate and pressurize the pressurized area, and the barometric pressure sensor (50) is configured to be connected with the other
air hole to collect a pressure signal in the pressurized area in real time, to obtain a pulse wave signal.

2. The blood pressure measurement apparatus according to claim 1, wherein the airbag (10) sequentially comprises a base section (104), an adjustable section (105), and a connection section (106) along the length direction of the airbag (10), the grooves are distributed on the adjustable section (105), a groove located at an edge of one side of the adjustable section (105) is a boundary between the adjustable section (105) and the base section (104), and a groove located at an edge of the other side of the adjustable section (105) is a boundary between the adjustable section (105) and the connection section (106).

3. The blood pressure measurement apparatus according to claim 2, wherein the grooves are distributed at equal intervals along a length direction of the adjustable section (105); or
the grooves are distributed along a length direction of the adjustable section (105) in a manner in which the grooves are sparse at two ends and dense in the middle.

4. The blood pressure measurement apparatus according to any one of claims 1 to 3, wherein a sealed cavity (109) is disposed at an inner side of the airbag (10) facing the air hole, the blocking part is disposed in the sealed cavity (109), an outer peripheral wall of the blocking part fits an inner peripheral wall of the sealed cavity (109), and a spring (111) is connected between the blocking part and the sealed cavity (109).

5. The blood pressure measurement apparatus according to claim 4, wherein the outer peripheral wall of the blocking part and the inner peripheral wall of the sealed cavity (109) are both in a serrated shape and form a concave-convex fit with each other.

6. The blood pressure measurement apparatus according to claim 1, wherein the pressing part (30) is plate-shaped, a first section of the groove is V-shaped, the first section is parallel to a length direction of the airbag (10), and is perpendicular to a width direction of the airbag (10), and the pressing part (30) can be pressed against a bottom of the groove from top to bottom, so that the airbag (10) is sealed by pressing at the groove to isolate air flow of the pressurized area from that of the non-pressurized area (115).

7. The blood pressure measurement apparatus according to claim 1, wherein a rotating shaft (60) is disposed in the cavity (20), the fixed end is fixed on the rotating shaft (60), and the rotating shaft (60) is rotatable to wind the airbag (10) around the rotating shaft (60).

8. The blood pressure measurement apparatus according to claim 7, wherein the cavity (20) comprises an accommodating part (201) and an extension part (203) connected to each other, an inner diameter of the accommodating part (201) is greater than an inner diameter of the extension part (203), the rotating shaft (60) is disposed in the accommodating part (201), the air pump (40) and the barometric pressure sensor (50) are disposed on an outer side of the extension part (203) and penetrate the extension part (203), and the pressing part (30) is disposed at a joint between the accommodating part (201) and the extension part (203) and functions as a gate of the accommodating part (201).

9. The blood pressure measurement apparatus according to claim 8, wherein in the length direction of the airbag (10), there is a first distance between a center of the pressing part (30) and a center of a first air nozzle (401) of the air pump (40) and a second distance between a center of the groove and a center of an adjacent air hole, and the first distance is equal to the second distance.

10. The blood pressure measurement apparatus according to claim 9, wherein the blood pressure measurement apparatus further comprises a fastener (70), the fastener (70) is disposed on the extension part (203), the airbag (10) penetrates the fastener (70), two positioning holes are disposed on a surface of the fastener (70), and the first air nozzle (401) of the air pump (40) and a second air nozzle of the barometric pressure sensor (50) may respectively pass through the two positioning holes to be inserted into corresponding air holes of the airbag (10).

11. The blood pressure measurement apparatus according to claim 10, wherein the blood pressure measurement apparatus further comprises a lifting platform configured to lift or lower down the fastener (70) and a corresponding part of the airbag (10).

12. The blood pressure measurement apparatus according to claim 11, wherein the blood pressure measurement apparatus further comprises a control board, a first driving part (100), and a second driving part (120), the first driving part (100) is configured to drive the rotating shaft (60) to rotate, the second driving part (120) is configured to drive the pressing part (30) to move, and the control board is separately electrically connected to the first driving part (100), the second driving part (120), the air pump (40), the barometric pressure sensor (50), and the lifting platform.

## Patentansprüche

1. Blutdruckmessvorrichtung, die eine Manschette (10), einen Hohlraum (20), ein Druckteil (30), eine Luftpumpe (40) und einen Luftdrucksensor (50) umfasst, wobei die Manschette (10) die Form eines langen Streifens hat und wobei die Manschette (10) umfasst:
eine Vielzahl von Rillen (101), wobei die Vielzahl von Rillen (101) auf einer Oberfläche der Manschette (10) entlang einer Längenerstreckungsrichtung der Manschette (10) verteilt ist, die Rillen sich entlang einer Breitenrichtung der Manschette (10) erstrecken, jede der Rillen konfiguriert ist, um die Manschette (10) entlang der Längenrichtung der Manschette (10) in einen unter Druck stehenden Bereich (110) und einen nicht unter Druck stehenden Bereich (115) zu unterteilen, wenn eine externe Struktur gegen eine Rille gedrückt wird, wobei der unter Druck stehende Bereich und der nicht unter Druck stehende Bereich (115) einen Luftstrom voneinander isolieren;
Luftlöcher (102), die zwischen jeweils zwei angrenzenden Rillen verteilt sind, wobei die Luftlöcher verwendet werden, um die Innenseite und die Außenseite des Airbags (10) zu verbinden; und
Sperrteile (103) zum Sperren der Luftlöcher,
wobei die Manschette (10) eine erste Oberfläche (107) und eine zweite Oberfläche (108) aufweist, die nacheinander angeordnet sind, die zweite Oberfläche (108) konfiguriert ist, um mit Haut in Kontakt zu stehen, jede Rille auf der ersten Oberfläche (107) angeordnet ist und jedes Luftloch auf der ersten Oberfläche (107) angeordnet ist,
**dadurch gekennzeichnet, dass** zwei Luftlöcher zwischen jeweils zwei angrenzenden Rillen verteilt sind, ein Luftloch konfiguriert ist, um mit der Luftpumpe (40) verbunden zu werden, das andere Luftloch konfiguriert ist, um mit dem Luftdrucksensor (50) verbunden zu werden, jede Rille auf der ersten Oberfläche (107) angeordnet ist, jedes Luftloch auf der ersten Oberfläche (107) angeordnet ist, und die zwei Luftlöcher zwischen zwei angrenzenden Rillen parallel entlang der Breitenrichtung der Manschette (10) angeordnet sind, wobei
die Manschette (10) ein befestigtes Ende und ein freies Ende aufweist, wobei das befestigte Ende in dem Hohlraum (20) befestigt ist und das freie Ende aus dem Hohlraum (20) herausgezogen werden kann;
das Druckteil (30) in dem Hohlraum (20) beweglich angeordnet ist und gegen die Rille gedrückt werden kann, um die Manschette (10) in einen unter Druck stehenden Bereich und einen nicht unter Druck stehenden Bereich (115) zu unterteilen, wobei der unter Druck stehende Bereich nahe dem freien Ende liegt; und
die Luftpumpe (40) konfiguriert ist, um mit dem einen Luftloch verbunden zu werden, um den unter Druck stehenden Bereich aufzublasen und unter Druck zu setzen, und der Luftdrucksensor (50) konfiguriert ist, um mit dem anderen Luftloch verbunden zu werden, um in Echtzeit ein Drucksignal in dem unter Druck stehenden Bereich zu erfassen, um ein Pulswellensignal zu erhalten.

2. Blutdruckmessvorrichtung nach Anspruch 1, wobei die Manschette (10) entlang der Längenrichtung der Manschette (10) der Reihe nach einen Basisabschnitt (104), einen einstellbaren Abschnitt (105) und einen Verbindungsabschnitt (106) umfasst, die Rillen auf dem einstellbaren Abschnitt (105) verteilt sind, eine an einer Kante einer Seite des einstellbaren Abschnitts (105) befindliche Rille eine Grenze zwischen dem einstellbaren Abschnitt (105) und dem Basisabschnitt (104) ist und eine an einer Kante der anderen Seite des einstellbaren Abschnitts (105) befindliche Rille eine Grenze zwischen dem einstellbaren Abschnitt (105) und dem Verbindungsabschnitt (106) ist.

3. Blutdruckmessvorrichtung nach Anspruch 2, wobei die Rillen in gleichmäßigen Intervallen entlang einer Längenrichtung des einstellbaren Abschnitts (105) verteilt sind; oder
die Rillen entlang einer Längenrichtung des einstellbaren Abschnitts (105) in einer Weise verteilt sind, in der die Rillen an zwei Enden dünner und in der Mitte dichter sind.

4. Blutdruckmessvorrichtung nach einem der Ansprüche 1 bis 3, wobei an einer Innenseite der Manschette (10) gegenüber dem Luftloch ein abgedichteter Hohlraum (109) angeordnet ist, das Sperrteil in dem abgedichteten Hohlraum (109) angeordnet ist, eine Außenumfangswand des Sperrteils an einer Innenumfangswand des abgedichteten Hohlraums (109) anliegt und eine Feder (111) zwischen dem Sperrteil und dem abgedichteten Hohlraum (109) verbunden ist.

5. Blutdruckmessvorrichtung nach Anspruch 4, wobei die Außenumfangswand des Sperrteils und die Innenumfangswand des abgedichteten Hohlraums (109) beide eine gezackte Form haben und eine konkav-konvexe Passung miteinander bilden.

6. Blutdruckmessvorrichtung nach Anspruch 1, wobei das Druckteil (30) plattenförmig ist, ein erster Abschnitt der Rille V-förmig ist, der erste Abschnitt parallel zu einer Längenrichtung der Manschette (10) ist und senkrecht zu einer Breitenrichtung der Manschette (10) ist und das Druckteil (30) von oben nach unten gegen einen Boden der Rille so gedrückt werden kann, dass die Manschette (10) durch Drücken an der Rille abgedichtet wird, um den Luftstrom des unter Druck stehenden Bereichs von dem des nicht unter Druck stehenden Bereichs (115) zu isolieren.

7. Blutdruckmessvorrichtung nach Anspruch 1, wobei eine Drehwelle (60) in dem Hohlraum (20) angeordnet ist, das befestigte Ende an der Drehwelle (60) befestigt ist und die Drehwelle (60) drehbar ist, um die Manschette (10) um die Drehwelle (60) zu wickeln.

8. Blutdruckmessvorrichtung nach Anspruch 7, wobei der Hohlraum (20) ein Aufnahmeteil (201) und ein Erstreckungsteil (203) umfasst, die miteinander verbunden sind, ein Innendurchmesser des Aufnahmeteils (201) größer als ein Innendurchmesser des Erstreckungsteils (203) ist, die Drehwelle (60) in dem Aufnahmeteil (201) angeordnet ist, die Luftpumpe (40) und der Luftdrucksensor (50) an einer Außenseite des Erstreckungsteils (203) angeordnet sind und das Erstreckungsteil (203) durchdringen, und das Druckteil (30) an einem Anschluss zwischen dem Aufnahmeteil (201) und dem Erstreckungsteil (203) angeordnet ist und als ein Tor des Aufnahmeteils (201) fungiert.

9. Blutdruckmessvorrichtung nach Anspruch 8, wobei in der Längenrichtung der Manschette (10) ein erster Abstand zwischen einer Mitte des Druckteils (30) und einer Mitte einer ersten Luftdüse (401) der Luftpumpe (40) und ein zweiter Abstand zwischen einer Mitte der Rille und einer Mitte eines angrenzenden Luftlochs vorhanden ist und der erste Abstand gleich dem zweiten Abstand ist.

10. Blutdruckmessvorrichtung nach Anspruch 9, wobei die Blutdruckmessvorrichtung ferner ein Anbringungselement (70) umfasst, das Anbringungselement (70) auf dem Erstreckungsteil (203) angeordnet ist, die Manschette (10) das Anbringungselement (70) durchdringt, zwei Positionierungslöcher auf einer Oberfläche des Anbringungselements (70) angeordnet sind und die erste Luftdüse (401) der Luftpumpe (40) und eine zweite Luftdüse des Luftdrucksensors (50) jeweils durch die zwei Positionierungslöcher hindurchgeführt werden können, um in entsprechende Luftlöcher der Manschette (10) eingeführt zu werden.

11. Blutdruckmessvorrichtung nach Anspruch 10, wobei die Blutdruckmessvorrichtung ferner eine Hebeplattform umfasst, die konfiguriert ist, um das Anbringungselement (70) und einen entsprechenden Teil der Manschette (10) anzuheben oder abzusenken.

12. Blutdruckmessvorrichtung nach Anspruch 11, wobei die Blutdruckmessvorrichtung ferner eine Steuerplatine, ein erstes Antriebsteil (100) und ein zweites Antriebsteil (120) umfasst, das erste Antriebsteil (100) konfiguriert ist, um die Drehwelle (60) anzutreiben, um sich zu drehen, das zweite Antriebsteil (120) konfiguriert ist, um das Druckteil (30) anzutreiben, um sich zu bewegen, und die Steuerplatine mit dem ersten Antriebsteil (100), dem zweiten Antriebsteil (120), der Luftpumpe (40), dem Luftdrucksensor (50) und der Hebeplattform separat elektrisch verbunden ist.

## Revendications

1. Appareil de mesure de pression artérielle comprenant un sac gonflable (10), une cavité (20), une partie pressante (30), une pompe à air (40) et un capteur de pression barométrique (50), dans lequel le sac gonflable (10) est en forme de longue bande, et dans lequel le sac gonflable (10) comprend :
une pluralité de rainures (101), la pluralité de rainures (101) sont réparties sur une surface du sac gonflable (10) dans le sens d'extension en longueur du sac gonflable (10), les rainures s'étendent le long d'un sens de la largeur du sac gonflable (10), chacune des rainures est conçue pour diviser le sac gonflable (10) en une zone pressurisée (110) et une zone non pressurisée (115) dans le sens de la longueur du sac gonflable (10) lorsqu'une structure externe est pressée contre une rainure, la zone pressurisée et la zone non pressurisée (115) isolent un flux d'air l'une de l'autre ;
des trous d'air (102) répartis entre deux rainures adjacentes, dans lequel les trous d'air sont utilisés pour relier l'intérieur et l'extérieur du sac gonflable (10) ; et
des parties de blocage (103) pour bloquer les trous d'air,
dans lequel le sac gonflable (10) a une première surface (107) et une seconde surface (108) qui sont disposées dos à dos, la seconde surface (108) est conçue pour être en contact avec la peau, chaque rainure est disposée sur la première surface (107), et chaque trou d'air est disposé sur la première surface (107),
**caractérisé en ce que** deux trous d'air sont répartis entre deux rainures adjacentes, un trou d'air est conçu pour être relié à la pompe à air (40), l'autre trou d'air est conçu pour être relié au capteur de pression barométrique (50), chaque rainure est disposée sur la première surface (107), chaque trou d'air est disposé sur la première surface (107), et les deux trous d'air entre deux rainures adjacentes sont disposés en parallèle dans le sens de la largeur du sac gonflable (10), dans lequel le sac gonflable (10) a une extrémité fixe et une extrémité libre, l'extrémité fixe est fixée dans la cavité (20), et l'extrémité libre peut être tirée hors de la cavité (20) ;
la partie pressante (30) est disposée de manière mobile dans la cavité (20) et peut être pressée contre la rainure pour diviser le sac gonflable (10) en une zone pressurisée et une zone non pressurisée (115), dans lequel la zone pressurisée est proche de l'extrémité libre ; et
la pompe à air (40) est conçue pour être reliée au trou d'air afin de gonfler et de pressuriser la zone pressurisée, et le capteur de pression barométrique (50) est conçu pour être relié à l'autre trou d'air afin de collecter un signal de pression dans la zone pressurisée en temps réel, afin d'obtenir un signal d'onde de pouls.

2. Appareil de mesure de pression artérielle selon la revendication 1, dans lequel le sac gonflable (10) comprend, de manière séquentielle, une section de base (104), une section ajustable (105), et une section de liaison (106) dans le sens de la longueur du sac gonflable (10), les rainures sont réparties sur la section ajustable (105), une rainure située au niveau d'un bord d'un côté de la section ajustable (105) constitue une limite entre la section ajustable (105) et la section de base (104), et une rainure située au niveau d'un bord de l'autre côté de la section ajustable (105) constitue une limite entre la section ajustable (105) et la section de liaison (106).

3. Appareil de mesure de pression artérielle selon la revendication 2, dans lequel les rainures sont réparties à intervalles égaux dans le sens de la longueur de la section ajustable (105) ; ou
les rainures sont réparties dans le sens de la longueur de la section ajustable (105) de manière que les rainures sont peu nombreuses au niveau de deux extrémités et denses au milieu.

4. Appareil de mesure de pression artérielle selon l'une quelconque des revendications 1 à 3, dans lequel une cavité scellée (109) est disposée au niveau d'un côté intérieur du sac gonflable (10) face au trou d'air, la partie de blocage est disposée dans la cavité scellée (109), une paroi périphérique extérieure de la partie de blocage épouse une paroi périphérique intérieure de la cavité scellée (109), et un ressort (111) est relié entre la partie de blocage et la cavité scellée (109).

5. Appareil de mesure de pression artérielle selon la revendication 4, dans lequel la paroi périphérique extérieure de la partie de blocage et la paroi périphérique intérieure de la cavité scellée (109) ont toutes deux une forme dentelée et forment un ajustement concave-convexe l'une par rapport à l'autre.

6. Appareil de mesure de pression artérielle selon la revendication 1, dans lequel la partie pressante (30) est en forme de plaque, une première section de la rainure est en forme de V, la première section est parallèle à un sens de la longueur du sac gonflable (10), et est perpendiculaire à un sens de la largeur du sac gonflable (10), et la partie pressante (30) peut être pressée contre un fond de la rainure de haut en bas, de sorte que le sac gonflable (10) est scellé en pressant au niveau de la rainure pour isoler un flux d'air de la zone pressurisée de celui de la zone non pressurisée (115).

7. Appareil de mesure de pression artérielle selon la revendication 1, dans lequel un arbre rotatif (60) est disposé dans la cavité (20), l'extrémité fixe est fixée sur l'arbre rotatif (60), et l'arbre rotatif (60) est rotatif pour enrouler le sac gonflable (10) autour de l'arbre rotatif (60).

8. Appareil de mesure de pression artérielle selon la revendication 7, dans lequel la cavité (20) comprend une partie de logement (201) et une partie d'extension (203) reliées l'une à l'autre, un diamètre intérieur de la partie de logement (201) est supérieur à un diamètre intérieur de la partie d'extension (203), l'arbre rotatif (60) est disposé dans la partie de logement (201), la pompe à air (40) et le capteur de pression barométrique (50) sont disposés sur un côté extérieur de la partie d'extension (203) et pénètrent dans la partie d'extension (203), et la partie pressante (30) est disposée au niveau d'une articulation entre la partie de logement (201) et la partie d'extension (203) et fonctionne en tant que porte de la partie de logement (201).

9. Appareil de mesure de pression artérielle selon la revendication 8, dans lequel, dans le sens de la longueur du sac gonflable (10), il existe une première distance entre un centre de la partie pressante (30) et un centre d'une première buse à air (401) de la pompe à air (40) et une seconde distance entre un centre de la rainure et un centre d'un trou d'air adjacent, et la première distance est égale à la seconde distance.

10. Appareil de mesure de pression artérielle selon la revendication 9, dans lequel l'appareil de mesure de pression artérielle comprend en outre une attache (70), l'attache (70) est disposée sur la partie d'extension (203), le sac gonflable (10) pénètre dans l'attache (70), deux trous de positionnement sont disposés sur une surface de l'attache (70), et la première buse à air (401) de la pompe à air (40) et une seconde buse à air du capteur de pression barométrique (50) peuvent respectivement passer à travers les deux trous de positionnement pour être insérées dans des trous d'air correspondants du sac gonflable (10).

11. Appareil de mesure de pression artérielle selon la revendication 10, dans lequel l'appareil de mesure de pression artérielle comprend en outre une plate-forme de levage conçue pour soulever ou abaisser l'attache (70) et une partie correspondante du sac gonflable (10).

12. Appareil de mesure de pression artérielle selon la revendication 11, dans lequel l'appareil de mesure de pression artérielle comprend en outre un tableau de commande, une première partie d'entraînement (100), et une seconde partie d'entraînement (120), la première partie d'entraînement (100) est conçue pour entraîner l'arbre rotatif (60) afin qu'il tourne, la seconde partie d'entraînement (120) est conçue pour entraîner la partie pressante (30) afin qu'elle se déplace, et le tableau de commande est électriquement connecté à la première partie d'entraînement (100), à la seconde partie d'entraînement (120), à la pompe à air (40), au capteur de pression barométrique (50), et à la plate-forme de levage séparément.
